# EUROPÄISCHE PATENTSCHRIFT

(11) **EP 1 596 856 B1**
(45) Veröffentlichungstag und Bekanntmachung des Hinweises auf die Patenterteilung: **13.12.2006**
(21) Anmeldenummer: 04708352.2
(22) Anmeldetag: 05.02.2004
(51) Int. Cl.: A61K 31/343, A61K 31/381, A61K 31/39, A61K 31/385, A61P 29/00, A61P 25/00, A61P 25/16, A61P 25/28

(54) **ARZNEIMITTEL ENTHALTEND SUBSTITUIERTE 2-ARYL-AMINOESSIGSÄURE-VERBINDUNGEN UND/ODER SUBSTITUIERTE 2-HETEROARYL-AMINOESSIGSÄURE-VERBINDUNGEN**
DRUGS CONTAINING SUBSTITUTED 2-ARYL-AMINOACETIC ACID COMPOUNDS AND/OR SUBSTITUTED 2-HETEROARYL-AMINOACETIC ACID COMPOUNDS
MEDICAMENTS CONTENANT DES ACIDES 2-ARYLAMINOACETIQUE SUBSTITUES ET/OU DES ACIDES 2-HETEROARYLAMINOACETIQUE SUBSTITUES

(30) Priorität: 13.02.2003 DE 10306202
(43) Veröffentlichungstag der Anmeldung: 23.11.2005
(73) Patentinhaber: Grünenthal GmbH, 52078 Aachen (DE)
(72) Erfinder: PRZEWOSNY, Michael, 52062 Aachen (DE); ENGLBERGER, Werner, 52223 Stolberg (DE); SUNDERMANN, Bernd, 52066 Aachen (DE); SCHIENE, Klaus, 40227 Düsseldorf (DE)
(74) Vertreter: Kutzenberger, Helga
(86) Internationale Anmeldenummer: PCT/EP2004/001062
(87) Internationale Veröffentlichungsnummer: WO 2004/071381

(56) Entgegenhaltungen:
- EP-A- 0 054 812
- EP-A- 0 568 136
- EP-A- 0 807 621
- EP-A- 1 078 917
- WO-A-00/35858
- WO-A-01/58875
- WO-A-01/90051
- WO-A-02/09688
- WO-A-02/44171
- WO-A-91/12002
- WO-A-98/11073
- WO-A-98/14427
- WO-A-98/22493
- WO-A-98/42673
- WO-A-03/059891
- US-A- 3 520 922
- US-A- 4 223 039
- US-B1- 6 277 850
- DATABASE WPI Section Ch, Week 197826 Derwent Publications Ltd., London, GB; Class B05, AN 1978-46269A XP002281201 & JP 53 015325 A (FUJISAWA PHARM CO LTD) 13. Februar 1978 (1978-02-13)

## Beschreibung

Die vorliegende Erfindung betrifft Arzneimittel enthaltend substituierte 2-Aryl-Aminoessigsäure-Verbindungen und/oder substituierte 2-Heteroaryl-Aminoessigsäure-Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, weisen jedoch unerwünschte Begleiterscheinungen wie beispielsweise Atemdepression, Erbrechen, Sedierung, Obstipation oder Toleranzentwicklung auf. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Protein-gekoppelten Rezeptoren gehören. Daneben gibt es weitere Rezeptoren und Ionenkanäle, die an dem System der Schmerzentstehung und Schmerzweiterleitung beteiligt sind, wie z. B. der N-Methyl-D-Aspartat (NMDA)-Ionenkanal, über den ein wesentlicher Teil der Kommunikation von Synapsen abläuft und durch den der Calcium-lonenaustausch zwischen einer neuronalen Zelle und ihrer Umgebung gesteuert wird.

Kenntnisse über die physiologische Bedeutung von lonenkanal-selektiven Substanzen sind durch die Entwicklung der patch-clamp-Technik gewonnen worden, mit der sich die Wirkung von NMDA-Antagonisten auf den Calciumhaushalt im Zellinneren nachweisen läßt.

Eine der vorliegenden Erfindung zugrundeliegenden Aufgabe war es daher, neue Arzneimittel zur Verfügung zu stellen, die sich insbesondere zur Bekämpfung von Schmerzen, vorzugsweise von chronischen und/oder neuropathischen Schmerzen eignen und die vorzugsweise die bei den Opioiden auftretenden unerwünschten Begleiterscheinungen nicht oder zumindest im verminderten Umfang zeigen.

Diese Aufgabe wurde durch Bereitstellung der erfindungsgemäßen Arzneimittel enthaltend wenigstens eine substituierte 2-Aryl-Aminoessigsäure-Verbindung und/oder wenigstens eine substituierte 2-Heteroaryl-Aminoessigsäure-Verbindung der nachstehenden allgemeinen Formel I gelöst.

Ein Gegenstand der vorliegenden Erfindung ist daher ein Arzneimittel enthaltend wenigstens eine substituierte 2-Aryl-Aminoessigsäure-Verbindung und/oder wenigstens eine substituierte 2-Heteroaryl-Aminoessigsäure-Verbindung der allgemeinen Formel I, 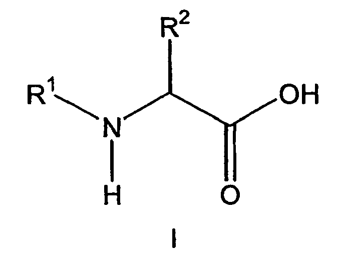 worin
R¹ für einen in 3,5-Position identisch substituierten Phenyl-Rest steht, wobei die Substituenten ausgewählt werden aus der Gruppe bestehend aus F, Cl, Br, I, OH, SH, unsubstituiertem C₁₋₆-Alkyl, unsubstituiertem C₁₋₆-Alkoxy, C₁₋₆-Perfluoralkyl, C₁₋₆-Perfluoralkoxy, unsubstituiertem C₁₋₆-Alkylsulfanyl, C₁₋₆-Perfluoralkylsulfanyl, unsubstituiertem Phenylsulfanyl, unsubstituiertem Cyclopentyl und unsubstituiertem Cyclohexyl.
und worin der rest R² ausgewählt ist aus der Gruppe bestehend aus Dibenzofuran-4-yl, Dibenzothiophen-4-yl, Phenoxathiin-4-yl, Thianthren-1-yl, 2-Methylsutfanylphenyl, 3-Methylsulfanylphenyl und 4-Methylsulfanylphenyl, besonders bevorzugt aus der Gruppe bestehend aus Dibenzofuran-4-yl, Dibenzothiophen-4-yl, Phenoxathiin-4-yl und Thianthren-1-yl, jeweils ggf. in Form eines ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate.

Sofern einer dieser vorstehend genannten Substituenten selbst einfach oder mehrfach substituiert ist, können dessen Substituenten vorzugsweise ausgewählt werden aus der Gruppe bestehend aus F, Cl, Br, Hydroxy, CN, CF₃, CHF₂ und CH₂F.

Ganz besonders bevorzugt enthalten die erfindungsgemäßen Arzneimittel wenigstens eine substituierte 2-Aryl-Aminoessigsäure-Verbindung und/oder wenigstens eine substituierte 2-Heteroaryl-Aminoessigsäure-Verbindung der allgemeinen Formel I ausgewählt aus der Gruppe bestehend aus:
(Dibenzofuran-4-yl)-(3,5-dichlorphenylamino)-essigsäure,
(Dibenzothiophen-4-yl)-(3,5-dichlorphenylamino)-essigsäure,
(3,5-Dichlorphenylamino)-(phenoxathiin-4-yl)-essigsäure,
(3,5-Dichlorphenylamino)-(thianthren-1-yl)-essigsäure,
(3,5-Dichlorphenylamino)-(2-methylsulfanylphenyl)-essigsäure,
(3,5-Dichlorphenylamino)-(4-methylsulfanylphenyl)-essigsäure und
(3,5-Bis(trifluormethyl)phenylamino)-(dibenzothiophen-4-yl)-essigsäure,
jeweils ggf. in Form eines ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate.

Es wurde überraschender Weise gefunden, daß die substituierten 2-Aryl-Aminoessigsäure-Verbindungen und/oder die substituierten 2-Heteroaryl-Aminoessigsäure-Verbindungen der vorstehenden allgemeinen Formel I eine hohe Affinität zur GlycinB-Bindungsstelle des NMDA-Rezeptorkanals aufweisen und sich als GlycinB-Antagonisten am NMDA Rezeptor Komplex zur Regulation des Calciumhaushalts der Zellen bei der Schmerzweiterleitung und damit unter anderem auch zur Regulation der Schmerzempfindung eignen.

Die erfindungsgemäßen Arzneimittel enthaltend wenigstens eine substituierte 2-Aryl-Aminoessigsäure-Verbindung und/oder wenigstens eine substituierte 2-Heteroaryl-Aminoessigsäure-Verbindung der allgemeinen Formel I zeigen insbesondere eine ausgeprägte Wirksamkeit bei der Bekämpfung von Schmerzen, vorzugsweise von chronischen und/oder neuropathischen Schmerzen.

Des weiteren eignen sich die erfindungsgemäßen Arzneimittel enthaltend wenigstens eine substituierte 2-Aryl-Aminoessigsäure-Verbindung und/oder wenigstens eine substituierte 2-Heteroaryl-Aminoessigsäure-Verbindung der allgemeinen Formel I zur Behandlung von neurodegenerativen Erkrankungen, vorzugsweise Morbus Alzheimer und/oder Morbus Huntington und/oder Morbus Parkinson und/oder multipler Sklerose.

Die Herstellung der erfindungsgemäß zum Einsatz kommenden substituierten 2-Aryl-Aminoessigsäure-Verbindungen sowie der substituierten 2-Heteroaryl-Aminoessigsäure-Verbindungen kann nach üblichen dem Fachmann bekannten Verfahren erfolgen, wie z.B. in US 6,232,467; WO 00/24510; WO 01/55091; N.A. Petasis, I.A. Zavialov, J. Am. Chem. Soc. 119, 445 - 446 (1997); N.A. Petasis, A. Goodman, I.A. Zavialov, Tetrahedron 53, 16463 - 16470 (1997), N.A. Petasis, S. Boral, Tetrahedron Lett. 42, 539 - 542 (2001) und G.S. Currie et al., J. Chem. Soc., Perkin Trans. 1 (2000), 2982 - 2990 beschrieben.

Vorzugsweise wird zur Herstellung der erfindungsgemäß zum Einsatz kommenden substituierten 2-Aryl-Aminoessigsäure-Verbindungen bzw. der substituierten 2-Heteroaryl-Aminoessigsäure-Verbindungen der allgemeinen Formel I wenigstens ein Amin der allgemeinen Formel II 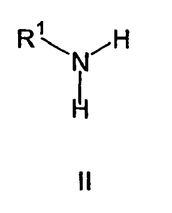 worin R¹ die vorstehend genannte Bedeutung hat, mit Glyoxylsäure (OHGCOOH), ggf. in Form eines Hydrates, und wenigstens einer Boronsäure der allgemeinen Formel III 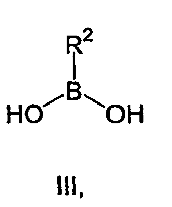 worin R² die vorstehend genannte Bedeutung hat, in einem geeigneten Reaktionsmedium, vorzugsweise in einem organischen Lösungsmittel, besonders bevorzugt in Methylenchlorid oder einem Gemisch enthaltend Methylenchlorid, zu wenigstens einer Verbindung der allgemeinen Formel I umgesetzt und diese ggf. nach üblichen, dem Fachmann bekannten Methoden gereinigt und isoliert.

Die vorstehend beschriebene Umsetzung erfolgt vorzugsweise bei einer Temperatur von 15°C bis 30°C, besonders bevorzugt bei einer Temperatur von 20 bis 25°C.

Das vorstehend beschriebene Verfahren zur Herstellung der in dem erfindungsgemäßen Arzneimittel zum Einsatz kommenden Verbindungen der allgemeinen Formel I hat den Vorteil, daß die eingesetzten Amine der allgemeinen Formel II, die Glyoxylsäure sowie die Boronsäuren der allgemeinen Formel III in dem Reaktionsmedium löslich, die substituierten 2-Aryl-Aminoessigsäure-Verbindungen sowie die substituierten 2-Heteroaryl-Aminoessigsäure-Verbindungen der allgemeinen Formel I dagegen üblicherweise unlöslich sind, so daß sie durch einfache Filtration und Waschen mit dem verwendeten Reaktionsmedium in reiner Form erhalten werden können.

Die substituierten 2-Aryl-Aminoessigsäure-Verbindungen und substituierten 2-Heteroaryl-Aminoessigsäure-Verbindungen der allgemeinen Formel I und entsprechende Stereoisomere können nach üblichen, dem Fachmann bekannten Verfahren in Form ihrer physiologisch verträglichen Salze erhalten werden, wobei die erfindungsgemäßen Arzneimittel eines oder mehrere Salze einer oder mehrerer Verbindungen der allgemeinen Formel I aufweisen können.

Sofern die erfindungsgemäß zum Einsatz kommenden Verbindungen der allgemeinen Formel I als basische Salze vorliegen, sind diese bevorzugt Salze der Alkalimetalle, Erdalkalimetalle oder Ammoniumsalze, besonders bevorzugt Natrium-, Kalium, Calcium, Magnesium oder Ammoniumsalze, wobei unter Ammoniumsalzen sowohl [NH₄]⁺ Salze als auch [NHₓR₄₋ₓ]⁺ Salze mit R = C₁₋₄-Alkyl und x = 0-3 verstanden werden, ganz besonders bevorzugt Natriumsalze.

Sofern die erfindungsgemäß zum Einsatz kommenden Verbindungen der allgemeinen Formel I als saure Salze vorliegen, können diese beispielsweise durch Umsetzung mit einer anorganischen oder organischen Säure, vorzugsweise mit Salzsäure, Bromwasserstoffsäure, Schwefelsäure, Methansulfonsäure, Ameisensäure, Essigsäure, Oxalsäure, Bemsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, Liponsäure, Acetylglycin, Acetylsalicylsäure, Hippursäure und/oder Asparaginsäure erhalten werden. Besonders bevorzugt sind die Hydrochlorid-Salze der erfindungsgemäß zum Einsatz kommenden Verbindungen der allgemeinen Formel I.

Die entsprechenden Hydrochloridsalze können vorzugsweise erhalten werden, indem die jeweilige substituierte 2-Aryl-Aminoessigsäure-Verbindung und/oder substituierte 2-Heteroaryl-Aminoessigsäure-Verbindung der allgemeinen Formel I und/oder entsprechende Stereoisomere durch Versetzen der in einem geeigneten organischen Lösungsmittel, wie z.B. Butan-2-on (Methylethylketon), gelösten Verbindungen der allgemeinen Formel I oder entsprechender Stereoisomere in unprotonierter Form mit Trimethylsilylchlorid (TMSCI) und Wasser in die entsprechenden Hydrochloridsalze übergeführt wird.

Die substituierten 2-Aryl-Aminoessigsäure-Verbindungen und/oder 2-Heteroaryl-Aminoessigsäure-Verbindungen der allgemeinen Formel I und entsprechende Stereoisomere können ggf. nach üblichen, dem Fachmann bekannten Verfahren, ebenso wie die entsprechenden Säuren, die entsprechenden Basen oder die Salze dieser Verbindungen, auch in Form ihrer Solvate, vorzugsweise ihrer Hydrate, erhalten werden.

Sofern die substituierten 2-Aryl-Aminoessigsäure-Verbindungen und/oder die substituierten 2-Heteroaryl-Aminoessigsäure-Verbindungen der allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Carnphersulfonsäure, gebildete diastereomere Salze voneinander getrennt werden.

Die substituierten 2-Aryl-Aminoessigsäure-Verbindungen und/oder 2-Heteroaryl-Aminoessigsäure-Verbindungen der allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist die Verwendung wenigstens einer substituierten 2-Aryl-Aminoessigsäure-Verbindung und/oder wenigstens einer substituierten 2-Heteroaryl-Aminoessigsäure-Verbindung der allgemeinen Formel I zur Herstellung eines Arzneimittels zur Bekämpfung von Schmerzen, vorzugsweise neuropathischen und/oder chronischen Schmerzen, sowie zur Behandlung von neurodegenerativen Erkrankungen, vorzugsweise von Morbus Alzheimer und/oder Morbus Huntington und/oder Morbus Parkinson und/oder multipler Sklerose.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einer oder mehreren substituierten 2-Aryl-Aminoessigsäure-Verbindungen und/oder einer oder mehreren substituierten 2-Heteroaryl-Aminoessigsäure-Verbindungen der allgemeinen Formel I, ggf. in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Substituierte 2-Aryl-Aminoessigsäure-Verbindungen und/oder substituierte 2-Heteroaryl-Aminoessigsäure-Verbindungen der allgemeinen Formel I, ggf. in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate, in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 2-Aryl-Aminoessigsäure-Verbindungen und/oder die substituierten 2-Heteroaryl-Aminoessigsäure-Verbindungen der allgemeinen Formel I, ggf. in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate auch verzögert freisetzen.

Die an den Patienten zu verabreichende Menge der jeweiligen substituierten 2-Aryl-Aminoessigsäure-Verbindungen und/oder der substituierten 2-Heteroaryl-Aminoessigsäure-Verbindungen der allgemeinen Formel I, ggf. in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer substituierten 2-Aryl-Aminoessigsäure-Verbindung und/oder wenigstens einer substituierten 2-Heteroaryl-Aminoessigsäure-Verbindung der allgemeinen Formel I, ggf. in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate appliziert.

### Pharmakologische Methoden

### (a) Rezeptorbindungs-Studie (GlycinB-Bindungsstelle des NMDA-Rezeptorkanals)

Die Bestimmung der Affinität der erfindungsgemäß zum Einsatz kommenden Verbindungen der allgemeinen Formel 1 zur Glycin-Bindungsstelle des NMDA-Rezeptorkanals werden an Himmembranhomogenaten (Homogenat von Cortex- und Hippocampus-Areal aus dem Hirn von männlichen Ratten, Stamm Wistar) durchgeführt, wie beispielsweise in B.M. Baron, B.W. Siegel, B.L. Harrison, R.S. Gross, C. Hawes and P. Towers, Journal of Pharmacology and Experimental Therapeutics, (1996), Vol. 279, S.62 beschrieben.

Hierzu werden Cortex und Hippocampus aus frisch entnommenen Rattengehimen freipräpariert und in 5 mmol/l TRIS-Acetatpuffer, 0,32 mol/l Saccharose pH 7,4 (10 ml/g Frischgewicht) mit einem Potter-Homogenisator (Fa. Braun/Melsungen; 10 Kolbenhübe bei 500 Upm) unter Eiskühlung homogenisiert und anschließend für 10 Minuten bei 1.000 g und 4°C zentrifugiert. Der erste Überstand wird gesammelt und das Sediment erneut mit 5 mmol/l TRIS-Acetatpuffer, 0,32 mol/l Saccharose pH 7,4 (5 ml/g ursprüngliches Frischgewicht) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wird mit dem Überstand aus der ersten Zentrifugation vereinigt und bei 17.000 g für 20 Minuten bei 4°C zentrifugiert. Der Überstand nach dieser Zentrifugation wird verworfen und das Membransediment mit 5 mmol/l TRIS-Acetatpuffer pH 8,0 (20 ml/g ursprüngliches Frischgewicht) aufgenommen und mit 10 Kolbenhüben bei 500 Upm homogenisiert.

Anschließend wird das Membranhomogenat für 1 Stunde bei 4°C inkubiert und für 30 Minuten bei 50.000 g und 4°C zentrifugiert. Der Überstand wird verworfen und das Zentrifugenröhrchen mit dem Membransediment mit Parafilm verschlossen und für 24 Stunden bei -20°C eingefroren. Am folgenden Tag wird das Membransediment aufgetaut und mit eiskaltem 5 mmol/l TRIS-Acetatpuffer, 0,1 % Saponin (Gewicht/Volumen) pH 7,0 (10 ml/g ursprüngliches Frischgewicht) aufgenommen und mit 10 Kolbenhüben bei 500 Upm homogenisiert und anschließend für 20 Minuten bei 50.000 g und 4°C zentrifugiert. Der resultierende Überstand wird verworfen und das Sediment in einem kleinen Volumen mit 5 mmol/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wird das Membranhomogenat mit 5 mmol/l TRIS-Acetatpuffer pH 7,0 auf eine Proteinkonzentration von 10 mg Protein/ml eingestellt und in Aliquoten bis zur Prüfung eingefroren.

Für den Rezeptorbindungstest werden Aliquote aufgetaut, 1:10 mit 5 mmol/l TRIS-Acetatpuffer pH 7,0 verdünnt, mit 10 Kolbenhüben bei 500 Upm mit dem Potter-Homogenisator (10 Kolbenhübe bei 500 Upm) unter Eiskühlung homogenisiert und für 60 Minuten bei 55.000 g bei 4°C zentrifugiert. Der Überstand wird dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat werden jeweils 100 µ je 1 ml-Ansatz im Rezeptorbindungstest eingesetzt (0,1 mg Protein/ml im Endansatz). Im Bindungstest wird als Puffer 50 mmol/l TRIS-Acetatpuffer pH 7,0 sowie als radioaktiver Ligand 1 nmol/l (³H)-MDL 105.519 (B.M. Baron et al. 1996) eingesetzt. Der Anteil an unspezifischer Bindung wird in Anwesenheit von 1 mmol/l Glycin bestimmt.

In weiteren Ansätzen werden die erfindungsgemäß zum Einsatz kommenden Verbindungen in Konzentrationsreihen zugegeben und die Verdrängung des radioaktiven Liganden aus seiner spezifischen Bindung an die Glycin-Bindungsstelle des NMDA-Rezeptorkanals ermittelt. Die jeweiligen Dreifachansätze werden über 120 Minuten bei 4°C inkubiert und anschließend zur Bestimmung des an das Membranhomogenat gebundenen radioaktiven Liganden mittels Filtration durch Glasfaser-Filtermatten (GF/B) geerntet. Die auf den Glasfaser-Filtern zurückgehaltene Radioaktivität wird nach Zugabe von Szintillator (Ready Protein, Fa. Beckmann Coulter GmbH, Krefeld, Deutschland) im β-Counter (Packard TRI-CARB Liquid Szintillation Analzer 2000 CA, Fa. Packard Instrument, Meriden, CT 06450, USA) gemessen.

### (b) Formalin-Test (Maus)

Die Untersuchungen zur Bestimmung der antinozizeptiven Wirkung der erfindungsgemäß zum Einsatz kommenden substituierten 2-Aryl-Aminoessigsäure-Verbindungen und/oder substituierten 2-Heteroaryl-Aminoessigsäure-Verbindungen werden im Formalin-Test an männlichen Albino-Mäusen (NMRI, 25 -35 g, Iffa, Credo, Belgien) durchgeführt.

Im Formalin-Test werden die erste (frühe) Phase (0-15 min nach Formalin-Injektion) und die zweite (späte) Phase (15-60 min nach Formalin-Injektion) unterschieden (D. Dubuisson et. Al., Pain, Vol. 4, pp. 161-174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al, Pain, Vol 52, pp. 259 - 285 (1993)).

Die erfindungsgemäß zum Einsatz kommenden Verbindungen der allgemeinen Formel I werden in der zweiten Phase des Formalin-Tests untersucht, um Aussagen über deren Wirkungen bei chronisch/entzündlichen Schmerz zu erhalten.

Durch eine einmalige subkutane Formalin-Injektion (20 µl, 1 Gew.-%ige wäßrige Lösung) in die dorsale Seite der rechten Hinterpfote wird bei freibeweglichen Versuchstieren eine nozizeptive Reaktion induziert, die sich in deutlichem Lecken und Beißen der betroffenen Pfote äußert.

Für den Untersuchungszeitraum in der zweiten (späten) Phase des Formalin-Tests wird das nozizeptive Verhalten durch Beobachtung der Tiere kontinuierlich erfaßt. Die Quantifizierung des Schmerzverhaltens erfolgt durch Summation der Sekunden, in denen die Tiere im Untersuchungszeitraum Lecken und Beißen der betroffenen Pfote zeigen. Nach Injektion von Verbindungen, die im Formalin-Test antinozizeptiv wirksam sind, sind die beschriebenen Verhaltensweisen der Tiere reduziert, evtl. sogar aufgehoben. Entsprechend den Versuchen, bei denen die Tiere die zu prüfende Verbindung vor Formalin injiziert bekommen, wird den Kontrolltieren Vehikel, d. h. Lösungsmittel (z. B. 0,9 Gew.-%ige NaCl-Lösung), vor der Formalinapplikation gespritzt. Das Verhalten der Tiere nach Gabe der zu prüfenden Verbindungen (n=10 pro Dosierung der Verbindung) wird mit einer Kontrollgruppe (n=10) verglichen. Basierend auf der Quantifizierung des Schmerzverhaltens wird die Wirkung im Formalin-Test als Änderung der Kontrolle in Prozent ermittelt. Die ED₅₀-Berechnungen erfolgt mittels Regressionsanalyse. Abhängig von der Applikationsart der erfindungsgemäß zum Einsatz kommenden Verbindungen wird der Applikationszeitpunkt vor der Formalin-Injektion gewählt (intraperitoneal: 15 min, intravenös: 5 min).

Im folgenden wird die Erfindung anhand von Beispielen erläutert.

### Beispiele

Die eingesetzten Chemikalien und Lösungsmittel wurden kommerziell bei den herkömmlichen Anbietem erworben (Acros, Aldrich, Chempur, Fluka, Lancaster und Merck).

Die NMR-Spektren wurden mit NMR-Spektrometern der Firma Bruker Analytik GmbH, Silberstreifen 4, D-76287 Rheinstetten gemessen. Die Gerätebezeichnungen lauten: für 300 MHz: Avance DPX 300 MHz, für 600 MHz: Avance DRX 600 MHz.

Die ESI-Massenspektren wurden mit einem Gerät vom Typ Finnigan LCQ der Firma Thermoquest (Analystische Systeme GmbH, Boschring 12, D-63329 Egelsbach) gemessen und mit der Software Xcalibur ausgewertet.

### Allgemeine Synthesevorschrift zur Herstellung der erfindungsgemäß zum Einsatz kommenden ggf. substituierten 2-Aryl-Aminoessigsäure-Verbindungen und/oder ggf. substituierten 2-Heteroaryl-Aminoessigsäure-Verbindungen der allgemeinen Formel I:

In 50 ml Dichlormethan wurden 10 mmol Glyoxylsäurehydrat gelöst, unter Rühren 10 mmol der jeweiligen Amin-Komponente der allgemeinen Formel II und 10 mmol der jeweiligen Boronsäurekomponente der allgemeinen Formel III zugegeben und über Nacht bei Raumtemperatur (ca. 20°C - 25°C) gerührt. Das ausgefallene Reaktionsprodukt wurde abgesaugt, mit wenig kaltem Dichlormethan gewaschen und anschließend im Hochvakuum getrocknet. Es wurden farblose Feststoffe erhalten.

Die jeweils hergestellte Verbindung der allgemeinen Formel I sowie die zu ihrer Herstellung eingesetzten Komponenten der allgemeinen Formeln II und III sind in der nachfolgenden Tabelle 1 wiedergegeben.

**Tabelle 1:**

| **Beispielverbindung** | **Aminkomponente der allgemeinen Formel II** | **Boronsäurekomponente der allgemeinen Formel III** |
|---|---|---|
| **Beispiel 1 :** | | |
| (Dibenzofuran-4-yl)-(3,5-dichlorphenylamino)-essigsäure | 3,5-Dichlorphenylamin | Dibenzofuran-4-yl-boronsäure |
| **Beispiel 2:** | 3,5-Dichlorphenylamin | Dibenzothiophen-4-yl- |
| (Dibenzothiophen-4-yl)-(3,5-dichlorphenylamino)-essigsäure | | boronsäure |
| **Beispiel 3:** | 3,5-Dichlorphenylamin | Phenoxathiin-4-yl- |
| (3,5-Dichlorphenylamino)-(phenoxathiin-4-yl)-essigsäure | | boronsäure |
| **Beispiel 4:** | 3,5-Dichlorphenylamin | Thianthren-1-yl- |
| (3,5-Dichlorphenylamino)-(thianthren-1-yl)-essigsäure | | boronsäure |
| **Beispiel 5:** | 3,5-Dichlorphenylamin | 2-Methylsulfanyl- |
| (3,5-Dichlorphenylamino)-(2-methylsulfanylphenyl)-essigsäure | | phenylboronsäure |
| **Beispiel 6:** | 3,5-Dichlorphenylamin | 4-Methylsulfanyl- |
| (3,5-Dichlorphenylamino)-(4 methylsulfanylphenyl)-essigsäure | | phenylboronsäure |
| **Beispiel 7:** | 3,5-bis-(Trifluormethyl)- | Dibenzothiophen-4-yl- |
| (3,5-Bis(trifluormethyl)-phenylamino)-(dibenzothiophen-4-yl)-essigsäure | phenylamin | boronsäure |

### Beispiel 1:

### (Dibenzofuran-4-yl)-(3,5-dichlorphenylamino)-essigsäure

Ausbeute: 3,86 g (100% der Theorie)
¹H-NMR (d₆-DMSOₑₓₜ): δ= 5,69 ppm (d, 1H, α-CH); 6,62 ppm (s,1H, Aryl-H); 6,73 ppm (s, 2H, 2 x Aryl-H); 7,08 ppm (d, 1H, α-NH); 7,43 ppm (m, 2H, 2 x Aryl-H); 7,56 ppm (m, 2H, 2 x Aryl-H); 7,77 ppm (d, 1H, J = 8,3 Hz, Aryl-H); 8,12 ppm (d, 1H, J = 7,6 Hz, Aryl-H); 8,16 ppm (d, 1H, J = 7,6 Hz, Aryl-H); 13,22 ppm (s (breit), 1H, CO₂H).
ESI-MS: Molmasse (berechnet für C₂₀H₁₃Cl₂NO₃): 386,20 g/mol
Gemessen (Positiv-Modus): 385,9 (MH⁺); 340,1 (M-CO₂).
Gemessen (Negativ-Modus): 383,9 (M-H); 340,3 (M-CO₂).

### Beispiel 2:

### (Dibenzothiophen-4-yl)-(3,5-dichlorphenylamino)-essigsäure

Ausbeute: 4,02 g (100% der Theorie)
¹H-NMR (d₆-DMSOₑₓₜ): δ = 5,44 ppm (d, 1H, α-CH); 6,60 ppm (s, 1H, Aryl-H); 6,70 ppm (s, 2H, 2 x Aryl-H); 7,11 ppm (d, 1H, α-NH); 7,50 ppm (m, 2H, 2 x Aryl-H); 7,57 ppm (m, 1H, Aryl-H); 7,67 ppm (m, 1H, Aryl-H); 8,03 ppm (m, 1H, Aryl-H); 8,38 ppm (m, 2H, 2 x Aryl-H); 13,38 ppm. (s (breit), 1H, CO₂H).
ESI-MS: Molmasse (berechnet für C₂₀H₁₃Cl₂NO₂S): 402,30 g/mol
Gemessen (Positiv-Modus): 401,8 (MH⁺); 356,1 (M-CO₂).
Gemessen (Negativ-Modus): 399,9 (M-H); 356,3 (M-CO₂).

### Beispiel 3:

### (3,5-Dichlorphenylamino)-(phenoxathiin-4-yl)-essigsäure

Ausbeute: 1,07 g (25,4% der Theorie)
¹H-NMR (d₆-DMSO_{ext.}): δ = 5,60 ppm (d, 1H, α-CH); 6,60 ppm (s, 1H, Aryl-H); 6,71 ppm (s, 2H, Aryl-H); 6,95 ppm (d, 1H, α-NH); 7,13 ppm (m, 2H, Aryl-H); 7,25 ppm (m, 1H, Aryl-H); 7,28 - 74,0 ppm (m, 5H, Aryl-H).
ESI-MS: Molmasse (berechnet für C₂₀H₁₃Cl₂NO₃S): 414,00 g/mol
Gemessen (Positiv-Modus): 417,8 (MH⁺).
Gemessen (Negativ-Modus): 416,1 (M-H); 372,4 (M-CO₂).

### Beispiel 4:

### (3,5-Dichlorphenylamino)-(thianthren-1-yl)-essigsäure

Ausbeute: 4,17 g (96% der Theorie)
¹H-NMR (d₆-DMSO_{ext.}): δ = 5,70 ppm (d, 1H, α-CH); 6,65 ppm (s, 2H, Aryl-H); 7,25 ppm (d, 1H, α-NH); 7,30 - 7,50 ppm (m, 3H, Aryl-H); 7,55 - 7,70 ppm (m, 2H, Aryl-H); 7,75 ppm (m, 1H, Aryl-H); 13,40 ppm (s (breit), 1H, CO₂H).
ESI-MS: Molmasse (berechnet für C₂₀H₁₃Cl₂NO₂S₂): 431,00 g/mol
Gemessen (Positiv-Modus): 433,8 (MH⁺).
Gemessen (Negativ-Modus): 434,0 (M-H); 388,3 (M-CO₂).

### Beispiel 5:

### (3,5-Dichlorphenylamino)-(2-methylsulfanylphenyl)-essigsäure

Ausbeute: 3,27 g (95,5 % der Theorie)
¹H-NMR (d₆-DMSO_{ext.}): δ = 2,55 ppm (s, 3H, SCH₃); 5,45 ppm (d, 1H, J = 6,8 Hz, α-CH); 6,60 ppm (m, 3H, 3 x Aryl-H); 7,03 ppm (d, 1H, J = 6,8 Hz, α-NH); 7,21 ppm (t, 1H, J = 6,8 Hz, Aryl-H): 7,35 ppm (m, 2H, J = 7,5-Hz, 2 x Aryl-H); 7,46 ppm (d, 1H, J = 7,6 Hz, Aryl-H); 13,11 ppm (s (breit), 1H, CO₂H).
ESI-MS: Molmasse (berechnet für C₁₅H₁₃Cl₂NO₂S): 342,25 g/mol
Gemessen (Positiv-Modus): 341,9 (MH⁺).
Gemessen (Negativ-Modus): 340,0 (M-H); 296,3 (M-CO₂).

### Beispiel 6:

### (3,5-Dichlorphenylamino)-(4-methylsulfanylphenyl)-essigsäure

Ausbeute: 2,96 g (86,5 % der Theorie)
¹H-NMR (d₆-DMSO_{ext.}): δ = 2,46 ppm (s, 3H, SCH₃); 5,16 ppm (d, 1H, α-CH); 6,60 ppm(s, 1H, Aryl-H); 6,67 ppm (s, 2H, Aryl-H); 6,90 ppm (d, 1H, α-NH); 7,26 ppm (d, 2H, J = 8,3 Hz, Aryl-H); 7,42 ppm (d, 2H, J = 8,3 Hz, Aryl-H); 13,04 ppm (s (breit), 1H, CO₂H).
ESI-MS: Molmasse (berechnet für C₁₅H₁₃Cl₂NO₂S): 342,25 g/mol
Gemessen (Negativ-Modus): 340,0 (M-H); 296,3 (M-CO₂).

### Beispiel 7:

### (3,5-Bis(trifluormethyl)phenylamino)-(dibenzothiophen-4-yl)-essigsäure

Ausbeute: 4,69 g (100% der Theorie)
¹H-NMR (d₆-DMSO_{ext.}): δ = 5,64 ppm (d, 1H, J = 5,7Hz, α-CH); 7,09 ppm (s, 1H, α-NH); 7,31 ppm (s, 2H, 2 x Aryl-H); 7,49 - 7,46 ppm (m, 4H, 4 x Aryl-H); 7,72 ppm (d, 1H, J = 7,1 Hz, Aryl-H); 8,04 ppm (m, 1H; Aryl-H); 8,35 - 8.41 ppm (m, 2H, 2 x Aryl-H); 13,49 ppm (s (breit), 1H, CO₂H).
ESI-MS: Molmasse (berechnet für C₂₂H₁₃F₆NO₂S): 469,41 g/mol
Gemessen (Negativ-Modus): 468,0 (M-H); 424,5 (M-CO₂).
Gemessen (Positiv-Modus): 469,8 (MH⁺); 424,3 (M-CO₂).

### Allgemeine Synthesevorschrift zur Herstellung der entsprechenden Natriumsalze der Verbindungen der allgemeinen Formel I:

10 mmol der jeweiligen substituierten 2-Aryl-Aminoessigsäure-Verbindung bzw. der jeweiligen substituierten 2-Heteroaryl-Aminoessigsäure-Verbindung der allgemeinen Formel I wurden in wenig Wasser suspendiert und 10 mmol 1-normaler wäßriger Natronlauge werden zugegeben. Bei schlechter Löslichkeit wurde soviel Methanol zugetropft, bis vollständige Lösung eintrat Nach 30 Minuten Rühren bei Raumtemperatur (ca. 20-25 °C) wurde die Lösung im Rotationsverdampfer eingeengt, die verbliebene Lösung bei -60°C in einem Gemisch aus Isopropanol/Trockeneis eingefroren und gefriergetrocknet. Die Natriumsalze wurden als farblose Feststoffe erhalten.

### Beispiel 8:

(Dibenzofuran-4-yl)-(3-5-dichlorphenylamino)-essigsäure, Natriumsalz
Ausbeute: 3,37 g (82,5 % der Theorie)
¹H-NMR (d₆-DMSO_{ext.}): δ = 5,64 ppm (d, 1H, α-CH); 6,60 ppm (s, 1H, Aryl-H); 6,71 ppm (s, 2H, 2 x Aryl-H); 7,06 ppm (d, 1H, J = 7,6 Hz, α-NH); 7,43 ppm (m, 2H, 2 x Aryl-H); 7,54 ppm (m, 2H, 2 x Aryl-H); 7,76 ppm (d, 1H, J = 8,3 Hz, Aryl-H); 8,10 (d, 1H, J = 7,6 Hz, Aryl-H); 8,16 ppm (d, 2H, J = 7,6 Hz, Aryl-H).

### Beispiel 9:

### (Dibenzothiophen-4-yl)-(3-5-dichlorphenylamino)-essigsäure, Natriumsalz

Ausbeute: 4,00 g (94% der Theorie)
¹H-NMR (d₆-DMSOₑₓₜ): δ = 4,70 ppm (m, 1H, α-CH); 6,45 ppm (s, 1H, Aryl-H); 6,49 ppm (s, 2H, 2 x Aryl-H); 6,82 ppm (m, 1H, α-NH); 7,44 ppm (m, 3H, 3 x Aryl-H); 7,56 ppm (m, 1H, Aryl-H); 7,96 ppm (m, 1H, J = 6,8 Hz, Aryl-H); 8,16 ppm (d, 1H, J = 7,6 Hz, Aryl-H).

### Beispiel 10:

### (3,5-Dichlorphenylamino)-(phenoxathiin-4-yl)-essigsäure, Natriumsalz

Ausbeute: 4,32 g (97,7 % der Theorie)
¹H-NMR (d₆-DMSO_{ext.}): δ = 4,99 ppm (d, 1H, J = 4,5 Hz, α-CH); 6,40 ppm (s, 1H, Aryl-H); 6,49 ppm (s, 2H, 2 x Aryl-H); 6,72 ppm (d, 1H, J = 5,3 Hz, α-NH); 6,97 ppm (m, 1H, Aryl-H); 7,05 ppm (m, 1H, Aryl-H); 7,12 ppm (m, 1H, Aryl-H), 7,17 ppm (m, 1H, Aryl-H); 7,25 - 7,32 ppm (m, 3H, 3 x Aryl-H).
ESI-MS: Molmasse (berechnet für C₂₀H₁₄Cl₂NNaO₃S): 443,0 g/mol
Gemessen (Negativ-Modus): 418,0 (M-H-Na); 372,3 (M-Na-CO₂).

### Beispiel 11:

(3,5-Dichlorphenylamino)-(thianthren-1yl)-essigsäure, Natriumsalz
Ausbeute: 4,18 g (87,2 % der Theorie)
¹H-NMR (d₆-DMSOₑₓₜ): δ = 5,07 ppm (s, 1H, α-CH); 6,33 ppm (s, 2H, 2 x Aryl-H); 6,38 ppm (s, 1H, Aryl-H); 6,84 ppm (d, 1H, J = 4,6Hz, α-NH); 7,18 ppm (t, 1H, J= 7,6 Hz, Aryl-H); 7,30 ppm (d, 1H, J = 7,6Hz, Aryl-H); 7,35 ppm (m, 2H, 2 x Aryl-H); 7.4,1 ppm (d, 1H, J = 7,6 Hz, Aryl-H); 7,58 ppm (m, 1H, Aryl-H); 7,69 ppm (m, 1H, Aryl-H).
ESI-MS: Molmasse (berechnet für C₂₀H₁₂Cl₂NNaO₂S₂); 479,33 g/mol
Gemessen (Negativ-Modus): 433,9 (M-H-Na); 388,4 (M-Na-CO₂).

### Beispiel 12:

### (3,5-Dichlorphenylamino)-(2-methylsulfanylphenyl)-essigsäure, Natriumsalz

Ausbeute: 3,56 g (97,9 % der Theorie)
¹H-NMR (d₆-DMSO_{ext.}): δ = 2,55 ppm (s, 3H, SCH₃); 4,95 ppm (m, 1H, α-CH); 6,41 ppm (s, 1H, Aryl-H); 6,47 ppm (s, 2H, 2 x Aryl-H); 6,77 ppm (d, 1H, J = 5,3 Hz, α-NH); 7,07 ppm (t, 1H, J = 7,5 Hz, Aryl-H); 7,15 ppm (t, 1H, J = 6,8 Hz, Aryl-H); 7,28 ppm (d, 1H, J = 7,6 Hz, Aryl-H); 7,36 ppm (d, 1H, J = 7,5 Hz, Aryl-H).

### Beispiel 13:

### (3,5-Dichlorphenylamino)-(4-methylsulfanylphenyl)-essigsäure, Natriumsalz

Ausbeute: 3,47 g (95,4 % der Theorie)
¹H-NMR (d₆-DMSO_{ext.}) δ = 2,43 ppm (s, 3H, SCH₃); 4,44 ppm (d, 1H, J = 4,59 Hz, α-CH); 6,46 ppm (m, 3H, 3 x Aryl-H); 6,64 ppm (d, 1H, J = 5,3 Hz, α-NH); 7,14 ppm (d, 2H, J = 8,3 Hz, Aryl-H); 7,34 ppm (d, 2H, J = 8,4 Hz, Aryl-H).

### Beispiel 14:

### (3,5-Bis(trifluormethyl)phenylamino)-(dibenzothiophen-4-yl)-essigsäure, Natriumsalz

Ausbeute: 4,91 g (100% der Theorie)
¹H-NMR (d₆-DMSOₑₓₜ.): δ = 4,74 ppm (s, 1H, α-CH); 6,88 ppm (s, 1H, Aryl-H); 7,07 ppm (s (breit), 2H, 2 x Aryl-H); 7,25 ppm (d, 1H, J = 3,7 Hz, α-NH); 7,45 ppm (m, 3H, 3 x Aryl-H); 7,57 ppm (d, 1H, J = 7,6 Hz, Aryl-H); 7,97 ppm (d, 1H, J = 6,8 Hz, Aryl-H); 8,16 ppm (d, 1H, J = 7,5 Hz, Aryl-H); 8,28 ppm (s, 1H, Aryl-H).
ESI-MS: Molmasse (berechnet für C₂₂H₁₂F₆NNaO₂S): 491,39 g/mol
Gemessen (Negativ-Modus): 467.9 (M-H-Na); 424,3 (M-Na-CO₂).

### Allgemeine Synthesevorschrift zur Herstellung der entsprechenden Hydrochloridsalze der Verbindungen der allgemeinen Formel I:

10 mmol der jeweiligen substituierten 2-Aryt-Aminoessigsäure-Verbindung bzw. der jeweiligen substituierten 2-Heteroaryl-Aminoessigsäure-Verbindung der allgemeinen Formel I wurden in 20 ml Butanon gelöst, unter Schutzgasatmosphäre, z. B. Stickstoff, in einem Eisbad abgekühlt, 10 mmol Trimethylsilychlorid und in einem Schuß 10 mmol Wasser zugegeben. Nach Rühren über Nacht wurde der ausgefallene Feststoff abfiltriert, mit wenig Butanon und anschließend mit Diethylether gewaschen. Die Hydrochloridsalze wurden als farblose Feststoffe erhalten.

### Beispiel 15:

### (Dibenzofuran-4-yl)-(3,5-dichlorphenylamino)-essigsäure, Hydrochlorid

Ausbeute: 0,93 g (2,2 % der Theorie)
¹H-NMR (d₆-DMSO_{ext.}): δ = 5,85 ppm (d, 1H, J = 4,5Hz, α-CH); 6,64 ppm (s, 1H, Aryl-H); 6,76 ppm (s, 2H, 2 x Aryl-H); 7,17 ppm (d, 1H, J = 8,4 Hz, α-NH); 7,43 ppm (t, 2H, J = 7,6 Hz, 2 x Aryl-H); 7,55 ppm (m, 2H, 2x Aryl-H); 7,76 ppm (d, 1H, J = 8,3 Hz, Aryl-H); 8,13 ppm (d, 1H, J = 7,5 Hz, Aryl-H); 8,16 ppm (d, 1H, J = 7,5 Hz, Aryl-H).
ESI-MS: Molmasse (berechnet für C₂₀H₁₄Cl₃NO₃): 422,70 g/mol
Gemessen (Negativ-Modus): 385,9 (M-HCl-H); 340,3 (M-CO₂).,

### Beispiel 16:

### (Dibenzothiophen-4-yl)-(3,5-dichlorphenylamino)-essigsäure, Hydrochorid

Ausbeute: 4,35 g (99,1 % der Theorie)
¹H-NMR (d₆-DMSO_{ext.}): δ = 5,47 ppm (d, 1H, J = 4,9 Hz, α-CH); 6,63 ppm (s, 1H, Aryl-H); 6.72 ppm (s, 2H, 2 x Aryl-H); 7,15 ppm (d, 1H, J = 5,6 Hz, α-NH); 7,48 - 7,58 ppm (m, 3 H, 3 x Aryl-H); 7,68 ppm (d, 1H, J = 6,8 Hz, Aryl-H); 8,04 ppm (m, 1H, Aryl-H); 8,36 ppm (m, 2H, 2 x Aryl-H); 13,41 ppm (s (breit), 1H, CO₂H).

### Beispiel 17:

### (Dichlorphenylamino)-(2-methylsulfanylphenyl)-essigsäure, Hydrochlorid

Ausbeute: 2,82 g (74,5 % der Theorie)
¹H-NMR (d₆-DMSOₑₓₜ): δ =2,54 ppm (s, 3H, SCH₃); 5,54 ppm (d, 1H, J =3,7 Hz, α-CH); 6,62 ppm (m, 2H, 2 x Aryl-H); 6,65 ppm (m, 1H, Aryl); 7,13 ppm (d, 1H, J = 7,5 Hz, α-NH); 7,22 ppm (t, 1H, J = Hz, 6,7 Hz, Aryl-H); 7,34 ppm (m, 1H, Aryl-H); 7,46 ppm (d, 1H, J = 6,7 Hz, Aryl-H).

### Beispiel 18:

### (3,5-Dichlorphenylamino)-(4-methylsulfanylphenyl)-essigsäure, Hydrochlorid

Ausbeute: 2,89 g (76,4 % der Theorie)
¹H-NMR (d₆-DMSO_{ext.}): δ = 2,46 ppm (s, 3H, SCH₃); 5,18 ppm (d, 1H, J = 5,6 Hz, α-CH); 6,62 ppm (t, 1H, J = 1,9 Hz, Aryl-H); 6,68 ppm (s, 2H, 2 x Aryl-H); 6,93 ppm (d, 1H, J = 7,2 Hz, α-NH); 7,26 ppm (d, 2H, J = 8,3 Hz, Aryl-H); 7,42 ppm (d, 2H, J = 8,2 Hz, Aryl-H).

### Pharmakologische Daten

### (a) Rezeptorbindungs-Studie (GlycinB-Bindungsstelle des NMDA-Rezeptorkanals)

Die Affinität der erfindungsgemäß zum Einsatz kommenden Verbindungen der allgemeinen Formel I zur Glydin-Bindungsstelle des NMDA-Rezeptorkanals wurde wie vorstehend beschrieben ermittelt und in der nachfolgenden Tabelle 2 als prozentualer Anteil des gebundenen radioaktiven Liganden angegeben, der bei einer Konzentration von 10 □M der zu prüfenden Verbindung der allgemeinen Formel I aus seiner spezifischen Bindung verdrängt wird, angegeben.

Die untersuchten Verbindungen der allgemeinen Formel I zeigten eine ausgezeichnete Affinität zur Glycin-Bindungsstelle des NMDA-Rezeptorkanals. Die Werte einiger ausgewählter Verbindungen sind in der nachfolgenden Tabelle 2 wiedergegeben.

**Tabelle 2:**

| **Verbindung gemäß Beispiel** | **Hemmung %** |
|---|---|
| 2 | 33 |
| 5 | 26 |
| 6 | 87 |
| 8 | 29 |
| 10 | 38 |
| 11 | 25 |
| 12 | 39 |
| 13 | 83 |

### (b) Formalin Test (Maus)

Die analgetische Wirkung der erfindungsgemäß zum Einsatz kommenden Verbindungen der allgemeinen Formel I wurde wie vorstehend beschrieben ermittelt. Die untersuchten Verbindungen zeigten jeweils eine mittelstarke bis starke Hemmung der durch Formalin induzierten Nozizeption.

In der nachfolgenden Tabelle 3 sind die Werte einiger ausgewählter Verbindungen wiedergegeben:

**Tabelle 3:**

| **Verbindung gemäß Beispiel** | **Hemmung (bei Dosis)** |
|---|---|
| 6 | 31% (bei 46,4 mg/kg) |
| 12 | 66% (bei 31,6 mg/kg) |

## Patentansprüche

1. Arzneimittel enthaltend wenigstens eine substituierte 2-Aryl-Aminoessig-säureVerbindung und/oder wenigstens eine substituierte 2-Heteroaryl-Aminoessigsäure-Verbindung der allgemeinen Formel I, 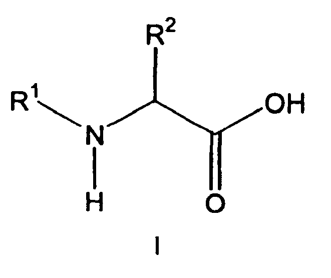 worin
R¹ für einen in 3,5-Position identisch substituierten Phenylrest steht, wobei die Substituenten ausgewählt sind aus der Gruppe bestehend aus F, Cl, Br, I, OH, SH, unsubstituiertem C₁₋₆-Alkyl, unsubstituiertem C₁₋₆-Alkoxy, C₁₋₆-Perfluoralkyl, C₁₋₆-Perfluoralkoxy, unsubstituiertem C₁₋₆-Alkylsulfanyl, C₁₋₆-Perfluoralkylsulfanyl, unsubstituiertem Phenylsulfanyl, unsubstituiertem Cyclopentyl und unsubstituiertem Cyclohexyl,
R² für einen Rest steht, der ausgewählt ist aus der Gruppe bestehend aus Dibenzofuran-4-yl, Dibenzothiophen-4-yl, Phenoxathiin-4-yl, Thianthren-1-yl, 2-Methylsulfanylphenyl, 3-Methylsulfanylphenyl und 4-Methylsulfanylphenyl,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate.

2. Arzneimittel gemäß Anspruch 1, **dadurch gekennzeichnet, daß** R² ausgewählt ist aus der Gruppe bestehend aus Dibenzofuran-4-yl, Dibenzothiophen-4-yl, Phenoxathün-4-yl und Thianthren-1-yl.

3. Arzneimittel gemäß Anspruch 1, **dadurch gekennzeichnet, daß** die Verbindung der allgemeinen Formel I ausgewählt ist aus der Gruppe bestehend aus:
(Dibenzofuran-4-yl)-(3,5-dichlorphenylamino)-essigsäure,
(Dibenzothiophen-4-yl)-(3,5-dichlorphenylamino)-essigsäure,
(3,5-Dichlorphenylamino)-(phenoxathiin-4-yl)-essigsäure,
(3,5-Dichlorphenylamino)-(thianthren-1-yl)-essigsäure,
(3,5-Dichlorphenylamino)-(2-methylsulfanylphenyl)-essigsäure,
(3,5-Dichlorphenylamino)-(4-methylsulfanylphenyl)-essigsäure und
(3,5-Bis(trifluormethyl)phenylamino)-(dibenzothiophen-4-yl)-essigsäure,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form ihrer Säuren oder ihrer Basen oder in Form ihrer physiologisch verträglichen Salze, insbesondere Natriumsalze oder Hydrochloridsalze, oder jeweils in Form ihrer Solvate, insbesondere Hydrate.

4. Arzneimittel gemäß einem der Ansprüche 1 - 3 zur Bekämpfung von Schmerzen

5. Arzneimittel gemäß Anspruch 4 zur Bekämpfung chronischer Schmerzen.

6. Arzneimittel gemäß Anspruch 4 zur Bekämpfung neuropathischer Schmerzen.

7. Arzneimittel gemäß einem der Ansprüche 1 - 3 zur Behandlung von neurodegenerativen Erkrankungen, vorzugsweise von Morbus Alzheimer und/oder Morbus Huntington und/oder Morbus Parkinson und/oder multipler Sklerose.

8. Verwendung wenigstens einer Verbindung gemäß einem der Ansprüche 1-3 zur Herstellung eines Arzneimittels zur Behandlung von Schmerzen, vorzugsweise von chronischen Schmerzen und/oder neuropathischen Schmerzen.

9. Verwendung wenigstens einer Verbindung gemäß einem der Ansprüche 1-3 zur Herstellung eines Arzneimittels zur Behandlung von neurodegenerativen Erkrankungen, vorzugsweise Morbus Alzheimer und/oder Morbus Huntington und/oder Morbus Parkinson und/oder multipler Sklerose.

## Claims

1. A pharmaceutical preparation containing at least one substituted 2-arylaminoacetic acid compound and/or at least one substituted 2-heteroarylaminoacetic acid compound of the general formula I, 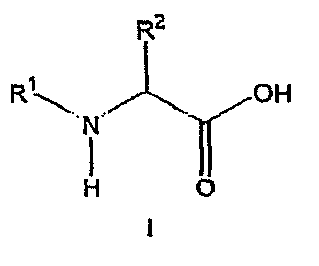 in which
R¹ denotes a phenyl residue which is identically substituted in 3,5-position, wherein the substituents are selected from the group consisting of F, Cl, Br, I, OH, SH, unsubstituted C₁₋₆ alkyl, unsubstituted C₁₋₆ alkoxy, C₁₋₆ perfluoroalkyl, C₁₋₆ perfluoroalkoxy, unsubstituted C₁₋₆ alkylsulfanyl, C₁₋₆ perfluoroalkylsulfanyl, unsubstituted phenylsulfanyl, unsubstituted cyclopentyl and unsubstituted cyclohexyl,
R² denotes a residue which is selected from the group consisting of dibenzofuran-4-yl, dibenzothiophen-4-yl, phenoxathiin-4-yl, thianthren-1-yl, 2-methylsulfanylphenyl, 3-methylsulfanylphenyl and 4-methylsulfanylphenyl,
in each case optionally in the form of one of the pure stereoisomers thereof, in particular enantiomers or diastereomers, the racemates thereof or in the form of a mixture of stereoisomers, in particular the enantiomers or diastereomers, in any desired mixing ratio, or in each case in the form of the acids thereof or the bases thereof or in the form of the physiologically acceptable salts thereof, in particular sodium salts or hydrochloride salts, or in each case in the form of the solvates thereof, in particular hydrates.

2. A pharmaceutical preparation according to claim 1, **characterised in that** R² is selected from the group consisting of dibenzofuran-4-yl, dibenzothiophen-4-yl, phenoxathiin-4-yl and thianthren-1-yl.

3. A pharmaceutical preparation according to claim 1, **characterised in that** the compound of the general formula I is selected from the group consisting of:
(dibenzofuran-4-yl)-(3,5-dichlorophenylamino)-acetic acid,
(dibenzothiophen-4-yl)-(3,5-dichlorophenylamino)-acetic acid,
(3,5-dichlorophenylamino)-(phenoxathiin-4-yl)-acetic acid,
(3,5-dichlorophenylamino)-(thianthren-1-yl)acetic acid,
(3,5-dichlorophenylamino)-(2-methylsulfanylphenyl)acetic acid,
(3,5-dichlorophenylamino)-(4-methylsulfanylphenyl)acetic acid and
(3,5-bis(trifluoromethyl)phenylamino)-(dibenzothiophen-4-yl)acetic acid,
in each case optionally in the form of one of the pure stereoisomers thereof, in particular enantiomers or diastereomers, the racemates thereof or in the form of a mixture of stereoisomers, in particular the enantiomers or diastereomers, in any desired mixing ratio, or in each case in the form of the acids thereof or the bases thereof or in the form of the physiologically acceptable salts thereof, in particular sodium salts or hydrochloride salts, or in each case in the form of the solvates thereof, in particular hydrates.

4. A pharmaceutical preparation according to any one of claims 1-3 for combatting pain.

5. A pharmaceutical preparation according to claim 4 for combatting chronic pain.

6. A pharmaceutical preparation according to claim 4 for combatting neuropathic pain.

7. A pharmaceutical preparation according to any one of claims 1-3 for the treatment of neurodegenerative diseases, preferably Alzheimer's disease and/or Huntington's chorea and/or Parkinson's disease and/or multiple sclerosis.

8. Use of at least one compound according to any one of claims 1-3 for the production of a pharmaceutical preparation for the treatment of pain, preferably of chronic pain and/or neuropathic pain.

9. Use of at least one compound according to any one of claims 1-3 for the production of a pharmaceutical preparation for the treatment of neurodegenerative diseases, preferably Alzheimer's disease and/or Huntington's chorea and/or Parkinson's disease and/or multiple sclerosis.

## Revendications

1. Médicament contenant au moins un composé de type 2-aryl-aminoacétique substitué et/ou au moins un composé de type 2-hétéroarylaminoacétique substitué de formule générale I, 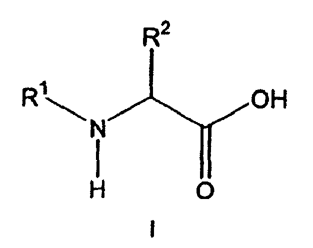 dans laquelle
R¹ représente un radical phényle substitué de façon identique en positions 3,5, les substituants étant choisis dans l'ensemble constitué par F, Cl, Br, I, OH, SH, des groupes alkyle en C₁-C₆ non substitués, alcoxy en C₁-C₆ non substitués, perfluoroalkyle (C₁-C₆), perfluoroalcoxy(C₁-C₆), alkyl(C₁-C₆)sulfanyle non substitué, perfluoroalkyl(C₁-C₆)sulfanyle, phénylsulfanyle non substitué, cyclopentyle non substitué et cyclohexyle non substitué,
R² représente un radical qui est choisi dans l'ensemble constitué par les groupes dibenzofurann-4-yle, dibenzothiophén-4-yle, phénoxathiyn-4-yle, thianthrén-1-yle, 2-méthylsulfanylphényle, 3-méthylsulfanylphényle et 4-méthylsulfanylphényle,
chaque fois éventuellement sous forme d'un de leurs stéréoisomères, en particulier énantiomères ou diastéréoisomères purs, de leurs racémates ou sous forme d'un mélange de stéréoisomères, en particulier des énantiomères ou diastéréoisomères, en un rapport quelconque de mélange, ou chaque fois sous forme de leurs acides ou de leurs bases ou sous forme de leurs sels physiologiquement acceptables, en particulier de leurs sels sodiques ou de leurs chlorhydrates, ou chaque fois sous forme de leurs produits de solvatation, en particulier d'hydrates.

2. Médicament selon la revendication 1, **caractérisé en ce que** R² est choisi dans l'ensemble consistant en les groupes dibenzofurann-4-yle, dibenzothiophén-4-yle, phénoxathiyn-4-yle et thianthrén-1-yle.

3. Médicament selon la revendication 1, **caractérisé en ce que** le composé de formule générale I est choisi dans le groupe constitué par :
l'acide (dibenzofurann-4-yl)-(3,5-dichlorophénylamino)acétique,
l'acide (dibenzothiophén-4-yl)-(3,5-dichlorophénylamino)acétique,
l'acide (3,5-dichlorophénylamino)-(phénoxathiyn-4-yl)acétique,
l'acide (3,5-dichlorophénylamino)-(thianthrén-1-yl)acétique,
l'acide (3,5-dichlorophénylamino)-(2-méthylsulfanylphényl)acétique,
l'acide (3,5-dichlorophénylamino)-(4-méthylsulfanylphényl)acétique et
l'acide (3,5-bis(trifluorométhyl)phénylamino)-(dibenzothiophén-4-yl)acétique,
chaque fois éventuellement sous forme d'un de leurs stéréoisomères, en particulier énantiomères ou diastéréoisomères purs, de leurs racémates ou sous forme d'un mélange de stéréoisomères, en particulier des énantiomères ou diastéréoisomères, en un rapport quelconque de mélange, ou chaque fois sous forme de leurs acides ou de leurs bases ou sous forme de leurs sels physiologiquement acceptables, en particulier de leurs sels sodiques ou de leurs chlorhydrates, ou chaque fois sous forme de leurs produits de solvatation, en particulier d'hydrates.

4. Médicament selon l'une quelconque des revendications 1 à 3, pour combattre des douleurs.

5. Médicament selon la revendication 4, pour combattre des douleurs chroniques.

6. Médicament selon la revendication 4, pour combattre des douleurs neuropathiques.

7. Médicament selon l'une quelconque des revendications 1 à 3, pour le traitement de maladies neurodégénératives, de préférence de la maladie d'Alzheimer et/ou de la chorée de Huntington et/ou de la maladie de Parkinson et/ou de la sclérose en plaques.

8. Utilisation d'au moins un composé selon l'une quelconque des revendications 1 à 3, pour la fabrication d'un médicament destiné au traitement de douleurs, de préférence de douleurs chroniques et/ou de douleurs neuropathiques.

9. Utilisation d'au moins un composé selon l'une quelconque des revendications 1 à 3, pour la fabrication d'un médicament destiné au traitement de maladies neurodégénératives, de préférence de la maladie d'Alzheimer et/ou de la chorée de Huntington et/ou de la maladie de Parkinson et/ou de la sclérose en plaques.
